(19) Europäisches Patentamt
European Patent Office
Office européen des brevets

(11) Publication number: **0 191 582**
**A1**

# (12) EUROPEAN PATENT APPLICATION

(21) Application number: **86300687.0**

(22) Date of filing: **31.01.86**

(51) Int. Cl.⁴: **C 07 D 249/20**, C 08 K 5/34, C 08 L 67/00, C 08 L 77/00 // C08J5/18

(30) Priority: **31.01.85 JP 18190/85**

(43) Date of publication of application: **20.08.86**
**Bulletin 86/34**

(84) Designated Contracting States: **DE FR GB IT**

(71) Applicant: **Takemoto Yushi Kabushiki Kaisha, 2-5, Minato-machi, Gamagouri-shi Aichi-ken (JP)**

(72) Inventor: **Ozaki, Tatsuhiko, 6-74 Eiraku-cho, Nishio-shi Aichi-ken (JP)**
Inventor: **Sugiura, Masato, 20-12 Matsubara-cho, Gamagouri-shi Aichi-ken (JP)**
Inventor: **Sugiura, Fumitoshi, 26-5 Takenoya-cho, Gamagouri-shi Aichi-ken (JP)**

(74) Representative: **Ablewhite, Alan James et al, MARKS & CLERK 57/60 Lincoln's Inn Fields, London WC2A 3LS (GB)**

## (54) Ultraviolet light absorbers of 2-phenylbenzotriazole type.

(57) New 2 phenylbenzotriazole derivatives of the general formula:

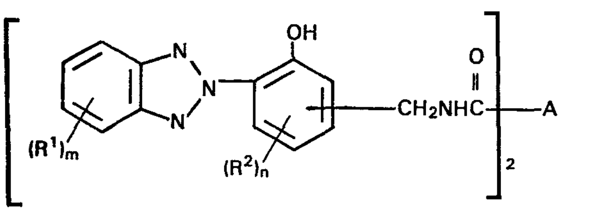

in which $R^1$ and $R^2$ are same or different and each represents a halogen atom, a hydroxy group, an alkyl group with 1-8 carbon atoms or an alkoxy group with 1-18 carbon atoms, A is a phenylene group, a halogen substituted phenylene group or a naphthylene group, m is zero or an integer in the range 1-4, n is zero or an integer in the range 1-3 and when m or n represents an integer of greater than 1 then the corresponding groups $R^1$ or $R^2$ are same or different. These compounds can be used as ultraviolet light absorbers which have superior characteristics regarding heat resistance and ability to absorb ultraviolet light.

EP 0 191 582 A1

M&C FOLIO: 799P51398 WANGDOC: 0160p

## ULTRAVIOLET LIGHT ABSORBERS OF 2-PHENYLBENZOTRIAZOLE TYPE

The present invention relates to a series of new 2-phenylbenzotriazole derivatives. These compounds may be used as ultraviolet light absorbers for improving weather resistance and more particularly the resistance of organic materials to light. Ultraviolet light absorbers of this type show improved thermal resistance.

Organic materials undergo rapid deterioration and decomposition when they are left outdoors. Sunlight is one of the major contributors to these phenomena. In particular, ultraviolet rays, with wavelengths in the range 290-400nm which are not absorbed by the atmosphere and reach the earth's surface, cause cleavage of covalent bonds in organic substances.

In order to protect organic materials from such ultraviolet rays, use has conventionally been made of compounds having the capability of strongly absorbing rays in this wavelength range and converting them into lower-energy rays and thermal energy which are harmless to organic materials. Such compounds may be called ultraviolet light absorbers and are either mixed with

the organic materials to be protected or applied on surfaces as a coating. Although many UV absorbers are known, for example:- 2-hydroxybenzophenone derivatives, 2 phenylbenzotriazole derivatives and salicylic acid derivatives, and have been used extensively in a variety of situations, most of them vaporize at relatively low temperatures or experience colouring or discolouring due to their low molecular weights and thermal instability. The ultraviolet light absorbers of the 2-phenylbenzotriazole type disclosed in British Patent No. 1,169,859 are said to have improved thermal resistance. Improvements to this British patent are disclosed in Japanese Patent Publication Tokko 56-5279, U.S. Patent No. 4,077,971 and West German Patent Publication No. 2,536,335, but these and the original British patents, are nor totally satisfactory regarding thermal resistance and in particular regarding the ability to absorb ultraviolet light rays.

Recently, furthermore, there has been a tendency to increase processing temperatures in order to speed up the moulding of synthetic resins and the drying/baking of coating materials. Moreover, synthetic resin materials such as polyamides, polyesters, polycarbonates, polyphenyl ethers, etc. which require moulding temperatures in excess of 250°C are becoming more frequently used. It is therefore strongly desirable to have available ultraviolet light absorbers

which have low volatility even at higher tempertures and do not colour or discolour, due to improved thermal resistance and have the capability of absorbing ultraviolet light beams.

In view of the above it is an object of the present invention to provide new ultraviolet light absorbers which can respond to the current requirements, eliminating the aforementioned drawbacks of conventional ultraviolet light absorbers.

More particularly, it is an object of the present invention to provide ultraviolet light absorbers which do not vaporize much even during a thermal process at temperatures in excess of 250°C, have sufficiently high thermal resistance so that there is no colouring or discolouring, and exhibit improved ability to absorb ultraviolet light beams.

The FIGURE is a graph showing the infrared spectrum of an ultraviolet light absorber embodying the present invention.

According to the present invention there is provided

a compound of the general formula:

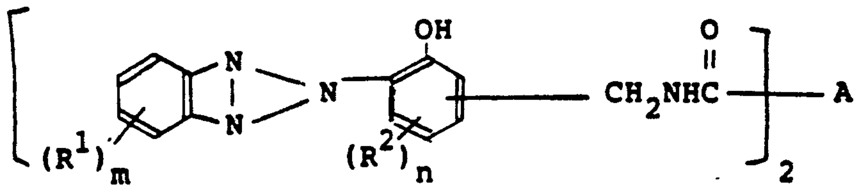

in which $R^1$ and $R^2$ are same or different and each represents a halogen atom, a hydroxy group, an alkyl group with 1-8 carbon atoms or an alkoxy group with 1-18 carbon atoms, A is a phenylene group, a halogen substituted phenylene group or a napthylene group, m is zero or an integer in the range 1-4, n is zero or an integer in the range 1-3 and when m or n represents an integer of greater than 1 then the corresponding groups $R^1$ or $R^2$ are same or different.

These compounds may be used as UV light absorbers when mixed with or applied to the surface of organic materials.

The compounds shown by the general formula I above (hereinafter referred to as the compounds of the present invention) are completely new compounds. They can be synthesized conveniently, for example, by the Tscherniac-Einhorn reaction (Organic Reaction, Vol. 14, p. 52, 1965 by John Wiley & Sons, Inc.) between 2-(2'-hydroxyphenyl)benzotriazole and N,N'-bis hydroxymethyl) aromatic dicarboxamide in the presence of an acid catalyst (such as a dehydrating agent like concentrated sulphuric acid). Examples of 2-(2'-hydroxyphenyl) benzotriazoles which can be used for synthesizing the compounds of the present invention include 2-(2'-hydroxy-5'-methylphenyl)benzotriazole, 2-(2'-hydroxy-5'-methylphenyl)-5-chlorobenzotriazole, 2-(2'-hydroxy-5'-t-butylphenyl)benzotriazole, 2-(2'-hydroxy-5'-t-butylphenyl)-5-chlorobenzotriazole, 2-(2'-hydroxy-5'-t-octylphenyl)benzotriazole, 2-(2'-hydroxy-4'-octoxyphenyl) benzotriazole, 2-(2'-hydroxy-3',5'-dimethylphenyl) benzotriazole, 2-(2'-hydroxy-3', 5'-dimethylphenyl)-5-chlorobenzotriazole 2-(2'-hydroxy-3',5',6'-trimethylphenyl) benzotriazole, 2-(2'-hydroxy-3',5',6'-trimethylphenyl)-5-chlorobenzotriazole, 2-(2'-hydroxy-4'-octoxy-5'-methylphenyl) benzotriazole-2-(2'-hydroxy-5'-methylphenyl)-5-chloro-6-methylbenzotriazole. Examples of N,N'-bis (hydroxymethyl) aromatic dicarboxamide which can be used for synthsizing the compounds of the present invention include N,N'-bis (hydroxymethyl)-1,3-benzenedicarboxamide and N,N'-bis (hydroxymethyl)-1,4-benzenedicarboxamide. They can be obtained easily by reacting isophthaloyl chloride or terephthaloyl chloride with ammonia and formaldehyde.

Eleven non-limiting examples of compounds of the present invention are shown below. Symbols used to

describe them have the same meanings as explained above. Structural formulae are given only for (I)-(III).

(I): m = 0, $(R^2)_n$ = $(CH_3)_1$, A = 1,3-phenylene

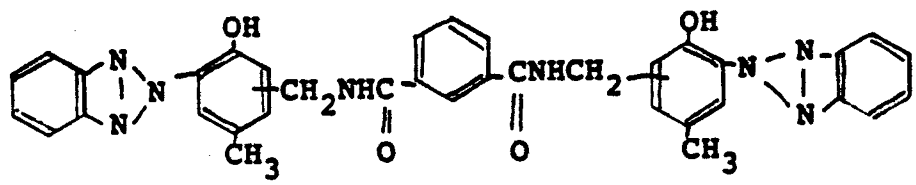

(II): m = 0, $(R^2)_n$ = $(CH_3)_1$, A = 1,4 - phenylene:

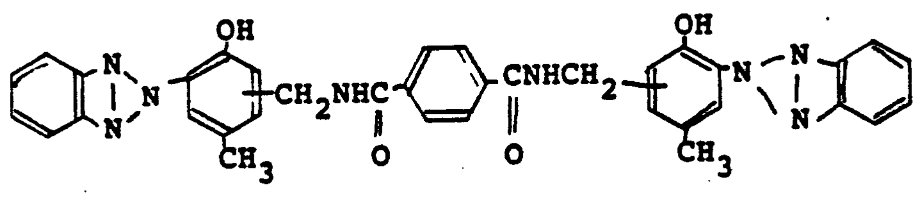

(III): m = 0, $(R^2)_n$ = $(OC_8H_{17})_1$, A = 1,3 - phenylene:

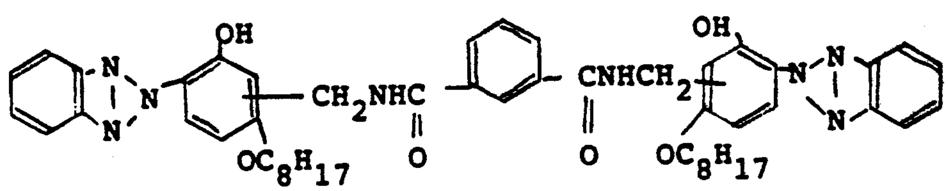

(IV): $(R^1)_m$ = $(CH_3)_1$+$(Cl)_1$, $(R^2)_n$ = $(t\text{-}C_4H_9)_1$, A = 1,3 - phenylene.

(V): m = 0, $(R^2)_n$ = $(t\text{-}C_4H_9)_1$, A = 1,4 - phenylene.

(VI): m = 0, $(R^2)_n$ = $(t\text{-}C_8H_{17})_1$, A = 1,3 - phenylene.

**(VII):** m = 0, $(R^2)_n$ = $(OH)_1$, A = 1,4 - phenylene.

**(VIII):** $(R^1)_m$ = $(Cl)_1$, $(R^2)_n$ = $(CH_3)_3$, A = 1,3 - phenylene.

**(IX):** $(R^1)_m$ = $(Cl)_1$, $(R^2)_n$ = $(CH_3)_3$, A = 1,4 - phenylene.

**(X):** m = 0, $(R^2)_n$ = $(CH_3)_1$, A = 2,6 - naphthalene.

**(XI):** m = 0, $(R^2)_n$ = $(CH_3)_1$, A = 2 - chloro - 1,4 - phenylene

The compounds of the present invention can be used in similar ways to conventional ultraviolet light absorbing agents. They can be mixed with organic macromolecule materials or used as a coating material on surfaces together with other substances. The amount used, is also similar to that of conventional absorbers. Although the optimum rate of application varies significantly, depending on the material to be protected, the purpose of protection, the method of application, the size and shape of the material to be protected, etc, it is usually about 0.5% when they are applied, for example, to polyethylene terephthalate resin.

The ultraviolet light absorbers of the present invention may be used in combination with other materials such as other ultraviolet light absorbers, antioxidative agents, antirust agents, and thermal stabilizers. A combined use with an antioxidative agent is particularly effective.

Since the compounds of the present invention are more heat resistant than the conventional absorbers, they can be used to provide weather resistance to materials which require high-temperature processing (in excess of 250°C) for manufacture for example polyethylene terephthalate, polyamides and polycarbonates. Moreover, they do not produce excessive amounts of smoke due to vaporization, nor is there the fear of contaminating apparatus such as moulds. There is no colouring or discolouring, and they exhibit improved ultraviolet light absorbing characteristics.

The following detailed examples are given to illustrate more clearly the present invention and its effects.

Example 1

45.0g of 2-(2'-Hydroxy-5'-methylphenyl)-benzotriazole were dissolved in 300g of 97% sulphuric acid

under nitrogen, and then cooled to 10°C with ice water. 22.4g of N,N'-bis(hydroxymethyl)-1,3-benzenedicarboxamide were added and the mixture was stirred for 40 hours at 20-30°C. The reactant was poured into two litres of cold water, and the deposited solid object was filtered and washed until it became neutral. The crude product was recrystalized from chloroform and 39.8g of slightly yellow solid (the aforementioned compound (I)) was obtained. Its melting point was 236-237°C and its infrared spectrum is shown in the Figure.

Example 2

Compound (II) was obtained by the method described in Example 1 but using appropriate starting materials.

Example 3

Compound (III) was obtained by the method described in Example I, but using appropriate starting materials.

Experiment 1

Regarding each of the aforementioned three compounds and three comparative examples to be described below, measurements were made to determine the wavelength at maximum absorption, the coefficient of molecular light

absorption and the temperature at which a weight loss starts due to vaporization. The results of this experiment are shown in Table 1.

TABLE 1

| | Maximum Absorption Wavelength (nm) | Coefficient of Molecular light Absorption | Temperature at which Weight loss Starts (°C) |
|---|---|---|---|
| Example | | | |
| 1 | 341 | $3.51 \times 10^4$ | 310 |
| 2 | 342 | $3.50 \times 10^4$ | 340 |
| 3 | 345 | $4.12 \times 10^4$ | 310 |
| Comparison | | | |
| 1 | 340 | $1.62 \times 10^4$ | 175 |
| 2 | 332 | $9.65 \times 10^3$ | 195 |
| 3 | 341 | $1.65 \times 10^4$ | 290 |

Coefficients of molecular light absorption were measured by using 1,1,2,2 - tetrachloroethane as solvent. The temperatures at which weight loss starts due to vaporization were measured by means of a thermo-balance as the temperature was raised at the rate of 10°C/min under nitrogen. The comparative examples Nos. 1 and 2 were respectively 2-(2'-hydroxy-5'-methylphenyl)-benzotriazole [Tinuvin P produced by CIBA-GEIGY, Inc - Trade Mark], and 2,4-dihydroxy benzophenone [Uvinul 400 produced by BASF, Inc - Trade Mark]. Comparative Example No.3 was a compound shown by the following formula

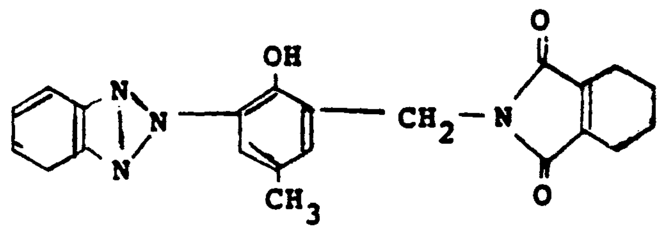

considered as an improvement on known uv light absorbers in Japanese Patent Publication Tokko 56-5279. The same comparative examples Nos. 1, 2 and 3 are used also in the following Experiments.

<u>Experiment 2</u>

0.5 Parts of each of the ultraviolet light absorbers shown in Table 2 were separately dry blended into 100 parts of 6-nylon chips. Each blended substance was melted and extruded from a T-die at 270°C by using a two-axis extruder to make a sheet of 0.5mm in thickness. Smoke generated during these processes was observed visually and recorded. The sheets were also irradiated for 100 hours with an ultraviolet beam by QUV and their yellowing was also observed visually and recorded. The results are shown in Table 2.

TABLE 2

| | Smoking | Yellowing |
|---|---|---|
| Example | | |
| 1 | None | None |
| 2 | None | None |
| 3 | None | None |
| Comparison | | |
| 0 | None | Yellowing |
| 1 | Extensive | Little |
| 2 | Extensive | Little |
| 3 | None | Slight |

In Table 2 (and also in Table 3 below), comparative example No.0 is a control without addition of any ultraviolet light absorber.

Experiment 3

0.5 Parts of each of the ultraviolet light absorbers shown in Table 3 were separately blended with 100 parts of polyethylene terephthalate chips of intrinsic viscosity 0.65. Undrawn films were obtained from these blended materials at 290°C by melting and extruding and

the smoke generated during the process was observed visually. Each of the undrawn films was thereafter subjected to a simultaneous two-axis drawing process with draw ratio of 3.5 x 3.5 at 80°C and made into a film of 0.125mm in thickness. This film was then irradiated continuously with an ultraviolet light beam by QUV and the time needed to reduce its impact strength to 20kg/cm was measured. The results of these experiments are shown in Table 3.

TABLE 3

| | Smoking | Time (hrs.) |
|---|---|---|
| Example | | |
| 1 | None | over 1000 |
| 2 | None | over 1000 |
| 3 | None | over 1000 |
| Comparison | | |
| 0 | None | 90 |
| 1 | Extensive | 450 |
| 2 | Extensive | 400 |
| 3 | Little | 800 |

Tables 1-3 clearly show the superior characteristics of the ultraviolet light absorbers of the present

invention regarding their heat resistance and ability to absorb ultraviolet light beams.

CLAIMS:

1. A compound of the general formula:

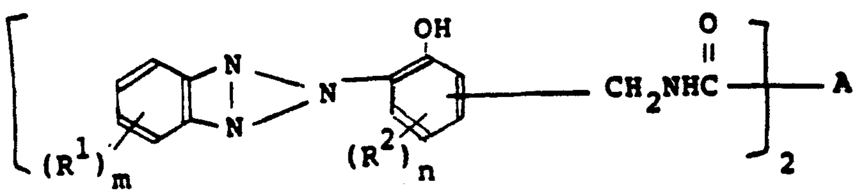

in which $R^1$ and $R^2$ are same or different and each represents a halogen atom, a hydroxy group, an alkyl group with 1-8 carbon atoms or an alkoxy group with 1-18 carbon atoms, A is a phenylene group, a halogen substituted phenylene group or a napthylene group, m is zero or an integer in the range 1-4, n is zero or an integer in the range 1-3 and when m or n represents an integer of greater than 1 then the corresponding groups $R^1$ or $R^2$ are same or different.

2. A compound as claimed in claim 1 wherein m=O.

3. A compound as claimed in claim 1 wherein n=1 or 3.

4. A compound as claimed in claim 1 wherein A is 1,3-phenylene, 1,4-phenylene, 2,6 napthalene or 2 chloro-1, 4-phenylene.

5. A compound of the formula I as defined in claim 1 selected from the following:-

(I): $m = 0$, $(R^2)_n = (CH_3)_1$, $A = 1,3$-phenylene

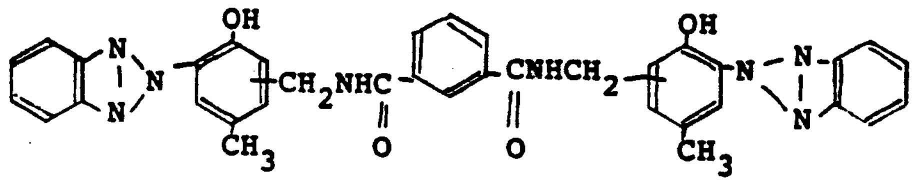

(II): $m = 0$, $(R^2)_n = (CH_3)_1$, $A = 1,4$ - phenylene:

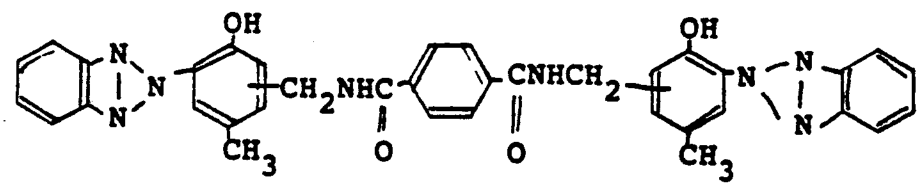

(III): $m = 0$, $(R^2)_n = (OC_8H_{17})_1$, $A = 1,3$ - phenylene:

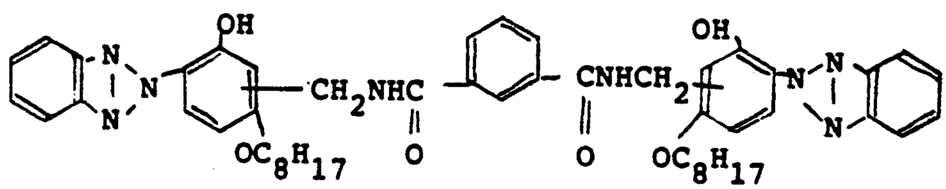

(IV): $(R^1)_m = (CH_3)_1 + (Cl)_1$, $(R^2)_n = (t\text{-}C_4H_9)_1$, $A = 1,3$ - phenylene.

(V): $m = 0$, $(R^2)_n = (t\text{-}C_4H_9)_1$, $A = 1,4$ - phenylene.

(VI): $m = 0$, $(R^2)_n = (t\text{-}C_8H_{17})_1$, $A = 1,3$ - phenylene.

**(VII):** m = 0, $(R^2)_n$ = $(OH)_1$, A = 1,4 - phenylene.

**(VIII):** $(R^1)_m$ = $(Cl)_1$, $(R^2)_n$ = $(CH_3)_3$, A = 1,3 - phenylene.

**(IX):** $(R^1)_m$ = $(Cl)_1$, $(R^2)_n$ = $(CH_3)_3$, A = 1,4 - phenylene.

**(X):** m = 0, $(R^2)_n$ = $(CH_3)_1$, A = 2,6 - naphthalene.

**(XI):** m = 0, $(R^2)_n$ = $(CH_3)_1$, A = 2 - chloro - 1,4 - phenylene

6. Organic material containing or coated with a compound of any of the preceding claims.

7. Polymeric materials containing or coated with a compound of any of the preceding claims.

8. Polyester or polyamide containing or coated with a compound of any of the preceding claims.

9. A method of protecting an organic material from uv degradation by incorporating therein a compound as claimed in any one of claims 1 to 5.

10. The use of a compound claimed in any of claims 1 to 5, as a uv absorber.

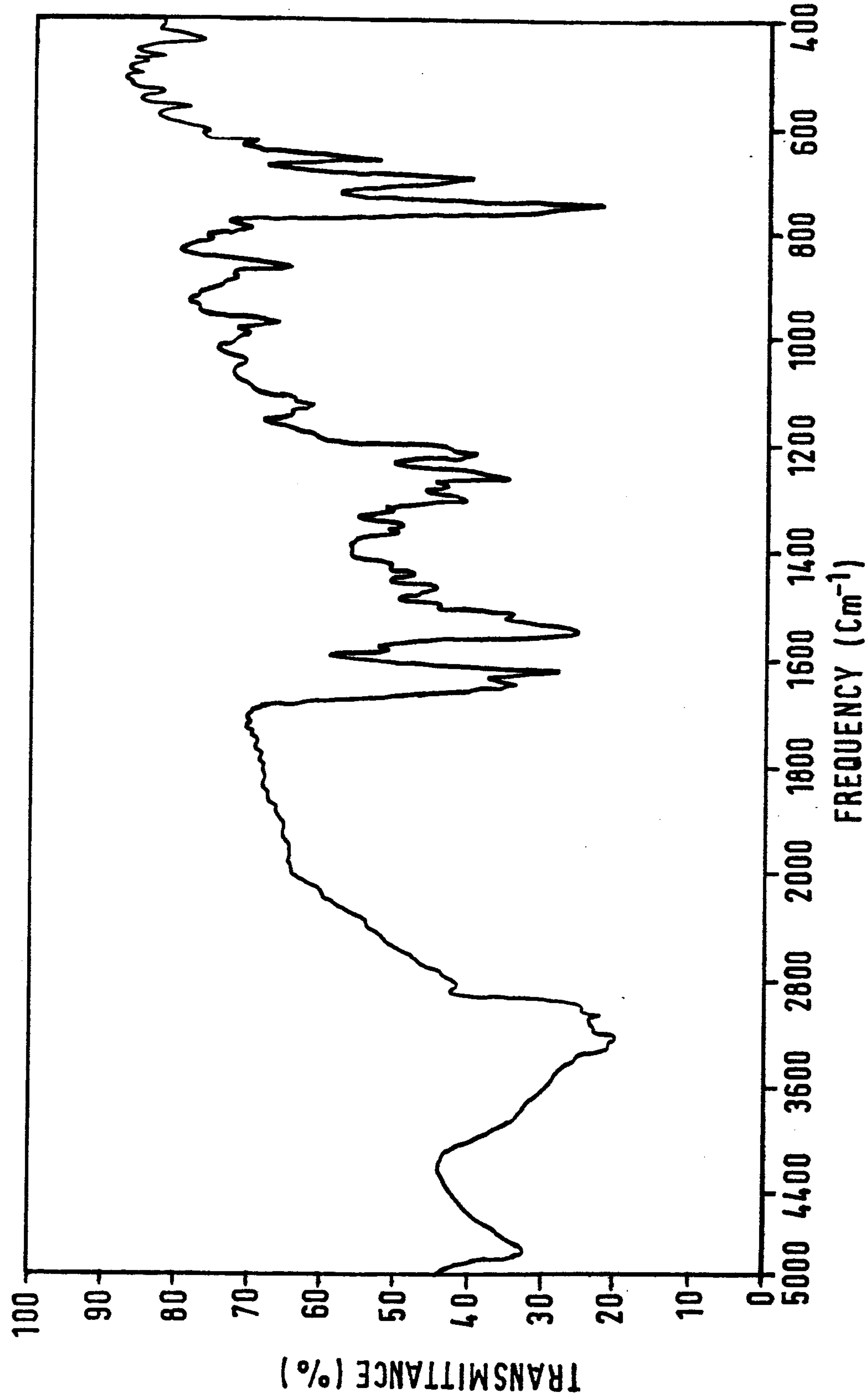
TRANSMITTANCE (%)
100
90
80
70
60
50
40
30
20
10
0
5000
4400
3600
2800
2000
1800
1600
1400
1200
1000
800
600
400
FREQUENCY (Cm$^{-1}$)

0191582

European Patent Office

# EUROPEAN SEARCH REPORT

Application number

EP 86 30 0687

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | GB-A- 991 204 (J.R. GEIGY A.-G.)<br>* page 1, line 9 - page 2, line 73, page 5, lines 17-95; examples 31, 32 *<br>--- | 1-10 | C 07 D 249/20<br>C 08 K 5/34<br>C 08 L 67/00<br>C 08 L 77/00 //<br>C 08 J 5/18 |
| Y | US-A-3 267 113 (R.A. CARBONI)<br>* column 1, lines 17-35, column 2, lines 1-16, 35-40, column 3, lines 8-22; column 23, lines 4-8; example 9 *<br>--- | 1-10 | |
| Y | DE-A-2 543 855 (EASTMAN KODAK CO.)<br>* claims 1, 7-10 *<br>--- | 1-10 | |
| A | PATENT ABSTRACTS OF JAPAN, vol. 2, no. 41 (C-77), 17th March 1978, page 4823 C 77; & JP - A - 52 147 649 (TOYO BOSEKI K.K.) 08-12-1977<br>--- | 1,6-10 | TECHNICAL FIELDS SEARCHED (Int. Cl.4)<br>C 07 D 249/00<br>C 08 K 5/00<br>C 08 L 67/00<br>C 08 L 77/00 |
| A,D | DE-A-2 536 335 (SUMITOMO CHEMICAL CO. LTD.)<br>* claims 1, 16, 17; examples 7, 12, 20, 21 * & JP - B - 56 5279; & US - A - 4 077 971 (Cat. D)<br>----- | 1,6-10 | |

The present search report has been drawn up for all claims

| Place of search | Date of completion of the search | Examiner |
|---|---|---|
| BERLIN | 20-04-1986 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82